# EUROPÄISCHE PATENTSCHRIFT

(11) **EP 0 657 171 B1**
(45) Veröffentlichungstag und Bekanntmachung des Hinweises auf die Patenterteilung: **12.05.1999**
(21) Anmeldenummer: 94118674.4
(22) Anmeldetag: 28.11.1994
(51) Int. Cl.: A61K 38/15

(54) **Endoparasitizide Mittel auf Basis von Offenkettigen Octadepsipeptiden**
Endoparasiticide drugs based upon open chain octadepsipeptides
Remèdes endoparasiticides à base de octadepsipeptides chaînes ouvertes

(30) Priorität: 09.12.1993 DE 4341993
(43) Veröffentlichungstag der Anmeldung: 14.06.1995
(73) Patentinhaber: BAYER AG, 51368 Leverkusen (DE)
(72) Erfinder: Scherkenbeck, Jürgen, Dr., D-42929 Wermelskirchen (DE); Jeschke, Peter, Dr., D-51373 Leverkusen (DE); Plant, Andrew, Dr., D-51519 Odenthal (DE); Harder, Achim, Dr., D-51109 Köln (DE); Mencke, Norbert, Dr., D-51381 Leverkusen (DE)

(56) Entgegenhaltungen:
- EP-A- 0 626 375
- EP-A- 0 626 376

## Beschreibung

Die vorliegende Erfindung betrifft die Verwendung von offenkettigen Octadepsipeptiden zur Herstellung von Mitteln zur Bekämpfung von Endoparasiten.

Bestimmte offenkettige Octadepsipeptide sind als Ausgangsstoffe für endoparasitizid wirksame cyclische Depsipeptide mit 24 Ringatomen Gegenstand vorgängiger, jedoch nicht vorveröffentlichter Patentanmeldungen (Deutsche Patentanmeldung P 4 317 457.4; P 4 317 432.9).

Über eine Verwendung dieser Verbindungen gegen Endoparasiten ist nichts bekannt.

Es wurde nun gefunden, daß die offenkettigen Octadepsipeptide der allgemeinen Formel (I) 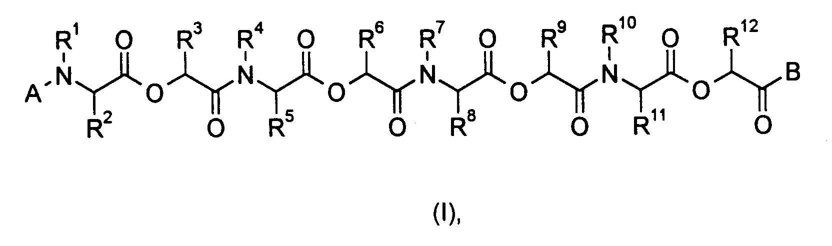 in welcher
- A: für Wasserstoff, Alkyl, Aralkyl oder einen Acylrest, insbesondere für einen Rest der Formel -CO-R¹⁵ steht, worin
- R¹⁵: für geradkettiges oder verzweigtes Alkyl, Alkenyl, Alkoxy, Aralkyl oder Arylalkoxy mit bis zu 6 Kohlenstoffatomen im Alkylteil steht,
- R¹, R⁴, R⁷ und R¹⁰: unabhängig voneinander für C₁-C₈-Alkyl, C₃-C₆-Cycloalkyl oder Aralkyl stehen,
- R², R⁵, R⁸ und R¹¹: unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie gegebenenfalls substituiertes Arylalkyl, wobei als Substituenten genannt seien Halogen, Hydroxy, Alkyl, Alkoxy stehen,
- R³, R⁶, R⁹ und R¹²: unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Alkylthioalkyl, Alkylsulfinylalkyl, Alkysulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Alkoxycarbonylaminoalkyl, Alkenyl, Cycloalkyl, Cycloalkylalkyl sowie gegebenenfalls substituiertes Aryl, Hetaryl oder Arylalkyl stehen, wobei als Substituenten genannt seien Halogen, Hydroxy, C₁-C₄-Alkyl, C₁-C₄-Alkoxy, Nitro oder eine Aminogruppe der Formel 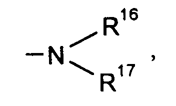 in der unabhängig voneinander jeder Rest R¹⁶ und R¹⁷ für Wasserstoff, Alkyl, Alkoxy steht oder beide Reste R¹⁶, R¹⁷ zusammen einen 5-, 6- oder 7-gliedrigen Ring bilden können, der gegebenenfalls durch O, S oder N unterbrochen ist und der gegebenenfalls mit C₁₋₄-Alkyl substituiert ist.
- B: für Hydroxy, Alkoxy mit bis zu 4 Kohlenstoffatomen oder den Rest NR¹³R¹⁴steht, wobei
R¹³ und R¹⁴ für Wasserstoff, Alkyl, Aralkyl oder Aryl stehen,
sowie deren optische Isomere und Racemate,
zur Herstellung von Mitteln zur Bekämpfung von Endoparasiten in der Medizin und Tiermedizin Verwendung finden können.

Die erfindungsgemäß zu verwendenden offenkettigen Octadepsipeptide sind durch die Formel (I) allgemein definiert.

Gegebenenfalls substituiertes Alkyl allein oder als Bestandteil eines Restes in den allgemeinen Formeln bedeutet geradkettiges oder verzweigtes Alkyl mit vorzugsweise 1 bis 9, insbesondere 1 bis 5 Kohlenstoffatomen. Beispielhaft und vorzugsweise seien gegebenenfalls substituiertes Methyl, Ethyl, n- und i-Propyl, n-, i- und t-Butyl, genannt.

Gegebenenfalls substituiertes Alkenyl allein oder als Bestandteil eines Restes in den allgemeinen Formeln bedeutet geradkettiges oder verzweigtes Alkenyl mit vorzugsweise 2 bis 20, insbesondere 2 bis 8 Kohlenstoffatomen. Beispielhaft und vorzugsweise seien gegebenenfalls substituiertes Ethenyl, Propenyl-(1), Propenyl-(2) und Butenyl-(3) genannt.

Gegebenenfalls substituiertes Cycloalkyl in den allgemeinen Formeln bedeutet mono-, bi- und tricyclisches Cycloalkyl mit vorzugsweise 3 bis 10, insbesondere 3, 5 oder 6 Kohlenstoffatomen. Beispielsweise und vorzugsweise seien gegebenenfalls substituiertes Cyclopropyl, Cyclobutyl, Cyclopentyl, Cyclohexyl und Cycloheptyl genannt.

Gegebenenfalls substituiertes Alkoxy in den allgemeinen Formeln bedeutet geradkettiges oder verzweigtes Alkoxy mit vorzugsweise 1 bis 6, insbesondere 1 bis 4 Kohlenstoffatomen. Beispielhaft und vorzugsweise seien gegebenenfalls substituiertes Methoxy, Ethoxy, n- und i-Propoxy und n-, s- und t-Butoxy genannt.

Gegebenenfalls substituiertes Alkylthio in den allgemeinen Formeln bedeutet geradkettiges oder verzweigtes Alkylthio mit vorzugsweise 1 bis 6, insbesondere 1 bis 4 Kohlenstoffatomen. Beispielhaft und vorzugsweise seien gegebenenfalls substituiertes Methylthio, Ethylthio, n- und i-Propylthio, n-, o- und t-Butylthio genannt.

Halogenalkyl in den allgemeinen Formeln enthält 1 bis 4, insbesondere 1 oder 22 Kohlenstoffatome und vorzugsweise 1 bis 9, insbesondere 1 bis 5 gleiche oder verschiedene Halogenatome, wobei als Halogenatome vorzugsweise Fluor, Chlor und Brom, insbesondere Fluor und Chlor stehen. Beispielhaft seien Trifluormethyl, Chlor-di-fluormethyl, 2,2,2-Trifluorethyl und Pentafluormethyl, Perfluor-t-butyl genannt.

Gegebenenfalls substituiertes Aryl in den allgemeinen Formeln bedeutet vorzugsweise gegebenenfalls substituiertes Phenyl oder Naphthyl, insbesondere Phenyl.

Gegebenenfalls substituiertes Arylalkyl in den allgemeinen Formeln bedeutet gegebenenfalls im Arylteil und/oder Alkylteil substituierte 6 Kohlenstoffatomen im Arylteil (vorzugsweise Phenyl oder Naphthyl, insbesondere Phenyl) und vorzugsweise 1 bis 4, insbesondere 1 oder 2 Kohlenstoffatomen im Alkylteil, wobei der Alkylteil geradkettig oder verzweigt sein kann. Beispielhaft und vorzugsweise seien gegebenenfalls substituiertes Benzyl und Phenylethyl genannt.

Gegegenenfalls Heteroaryl allein oder als Bestandteil eines Restes bedeutet in den allgemeinen Formeln 5- bis 7-gliedrige Ringe mit vorzugsweise 1 bis 3, insbesondere 1 oder 2 gleichen oder verschiedenen Heteroatomen. Als Heteroatome stehen Sauerstoff, Schwefel oder Stickstoff. Beispielhaft und vorzugsweise seien gegebenenfalls substituiertes Furyl, Thienyl, Pyrazolyl, Imidazolyl, 1,2,3-und 1,2,4-Triazolyl, Oxazolyl, Isoxazolyl, Thiazolyl, Isothiazolyl, 1,2,3-, 1,3,4-, 1,2,4- und 1,2,5-Oxadiazolyl, Azepinyl, Pyrrolyl, Isopyrrolyl, Pyridyl, Piperazinyl, Pyridazinyl, Pyrimidinyl, Pyrazinyl, 1,3,5-, 1,2,4- und 1,2,3-Triazinyl, 1,2,4-, 1,2,3-, 1,3,6- und 1,2,6-Oxazinyl, Oxepinyl, Thiepinyl und 1,2,4-Diazepinyl genannt.

Die gegebenenfalls substituierten Reste der allgemeinen Formel können einen oder mehrere, vorzugsweise 1 bis 3, insbesondere 1 bis 2 gleiche oder verschiedene Substituenten tragen. Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethlythio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Difluormethyl, Trifluormethyl; Hydroxy; Halogen, vorzugsweise Fluor, Chlor, Brom, insbesondere Fluor, Chlor, Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methylethylamino, n- und i-Propylamino und Methyl-n-Butylamino; Acrylreste wie Carboxyl; Carboxy mit vorzugsweise 2 bis 4, insbesondere 2 oder 3 Kohlenstoffatomen, wie Carbomethoxy und Carboethoxy; Alkylsulfinyl mit 1 bis 4, insbesondere 1 bis 2 Kohlenstoffatomen, Halogenalkylsulfinyl mit 1 bis 4, insbesondere 1 bis 2 Kohlenstoffatomen und 1 bis 5 Halogenatomen wie Trifluormethylsulfinyl; Sulfonyl (-SO₃H); Alkylsulfonyl mit vorzugsweise 1 bis 4, insbesondere 1 oder 2 Kohlenstoffatomen, wie Methylsulfonyl und Ethyllsulfonyl; Halogenalkylsulfonyl mit 1 bis 4, insbesondere 1 bis 2 Kohlenstoffatomen und 1 bis 5 Halogenatomen wie Trifluormethylsulfonyl, Perfluor-t,n,s-butylsulfonyl; Arylsulfonyl mit vorzugsweise 6 oder 10 Arylkohlenstoffatomen, wie Phenyllsulfonyl; Acyl, Aryl, Aryloxy, Heteroaryl, Heteroaryloxy, die ihrerseits einen der oben genannten Substituenten tragen können sowie der Formiminorest

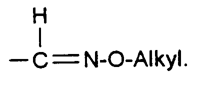

Vorzugsweise werden erfindungsgemäß diejenigen Verbindungen der Formel (I) verwendet
in welcher
- A: für Wasserstoff, C₁-C₄-Alkyl oder Benzyl steht,
oder für eine Gruppe der Formel -CO-R¹⁵ steht,
worin
- R¹⁵: für geradkettiges oder verzweigtes C₁-C₄-Alkyl, C₂-C₄-Alkenyl, C₁-C₄-Alkoxy oder Phenylalkoxy mit bis zu 6 Kohlenstoffatomen im Alkylteil, insbesondere für tert.-Butoxy, Benzyloxy, Ethoxy, Allyloxy, Fluorenyl-9-methoxy oder Methoxy steht,
- R¹, R⁴, R⁷ und R¹⁰: unabhängig voneinander für C₁-C₄-Alkyl, C₃-C₆-Cycloalkyl, Phenyl-C₁₋₄-alkyl stehen,
- R², R⁵, R⁸ und R¹¹: unabhängig voneinander für Wasserstoff, geradkettiges oder verzweigtes C₁₋₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Octyl, Isooctyl, sec.Octyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, Guanido-C₁-C₆-alkyl, insbesondere Guanidopropyl, C₁-C₄-Alkoxycarbonylamino-C₁-C₆-alkyl, insbesondere tert.-Butoxycarbonylaminopropyl, tert.-Butoxycarbonylaminobutyl, 9-Fluorenylmethoxycarbonyl(Fmoc)aminopropyl, 9-Fluorenylmethoxycarbonyl(Fmoc)aminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl, das gegebenenfalls durch Reste aus der Reihe Halogen, insbesondere Fluor, Chlor, Brom oder Iod, Hydroxy, C₁-C₄-Alkoxy, insbesondere Methoxy oder Ethoxy, C₁-C₄-Alkyl, insbesondere Methyl, substituiert sein kann stehen,
- R³, R⁶, R⁹ und R¹²: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Octyl, Isooctyl, sec.-Octyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, Guanido-C₁-C₆-alkyl, insbesondere Guanidopropyl, C₁-C₄-Alkoxycarbonylamino-C₁-C₆-alkyl, insbesondere tert.Butoxycarbonylaminopropyl, tert.-Butoxycarbonylaminobutyl, 9-Fluorenylmethoxycarbonyl-(Fmoc)aminopropyl, 9-Fluorenylmethoxycarbonyl(Fmoc)-aminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Aryl-C₁-C₄-alkyl oder Hetaryl-C₁-C₄-alkyl, die gegebenenfalls durch Reste substituiert sein können aus der Reihe Halogen, insbesondere Fluor, Chlor, Brom oder Iod, Hydroxy, C₁-C₄-Alkoxy, insbesondere Methoxy oder Ethoxy, C₁-C₄-Alkyl, insbesondere Methyl, Nitro oder eine Aminogruppe der Formel 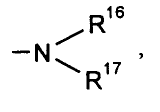 in der jeder Rest R¹⁶ oder R¹⁷ für Wasserstoff, C₁₋₄-Alkyl, C₁₋₄-Alkoxy oder beide Reste zusammen einen 5-, 6- oder 7-gliedrigen Ring bilden können, der gegebenenfalls durch O, S oder N unterbrochen ist und der gegebenenfalls mit C₁-C₄-Alkyl substituiert ist.
- B: für Hydroxy, tert.-Butoxy oder den Rest NR¹³R¹⁴ steht, wobei
- R¹³ und R¹⁴: unabhängig voneinander für Wasserstoff, geradkettiges oder verzweigtes C₁₋₄-Alkyl, insbesondere Methyl, Ethyl, Isopropyl, t-Butyl, Benzyl, Cyclopropyl, Cyclohexyl, Phenyl stehen
sowie deren optische Isomere und Racemate.

Besonders bevorzugt werden erfindungsgemäß diejenigen Verbindungen der Formel (I) verwendet
in welcher
- A: für Wasserstoff, Benzyl oder eine Gruppe COR¹⁵ steht,
- R¹⁵: für geradkettiges oder verzweigtes Alkoxy oder Arylalkoxy mit bis zu 6 Kohlenstoffatomen im Alkylteil, insbesondere für tert.-Butoxy, Benzyloxy steht,
- R¹, R⁴, R⁷ und R¹⁰: unabhängig voneinander für C₁₋₄-Alkyl, C₃₋₆-Cycloalkyl und Benzyl stehen,
- R², R⁵, R⁸ und R¹¹: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, Hydroxy-C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, C₁-C₄-Alkoxycarbonylamino-C₁-C₆-alkyl, insbesondere tert.-Butoxycarbonylaminopropyl, tert.-Butoxycarbonylaminobutyl, C₂-C₈-Alkenyl, insbesondere Vinyl, Allyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl, das gegebenenfalls durch einen oder mehrere gleiche oder verschiedene der oben angegebenen Reste substituiert sein kann stehen,
- R³, R⁶, R⁹ und R¹²: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, Hydroxy-C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, C₁-C₄-Alkoxycarbonylamino-C₁-C₆-alkyl, insbesondere tert.-Butoxycarbonylaminopropyl, tert.-Butoxycarbonylaminobutyl, C₂-C₈-Alkenyl, insbesondere Vinyl, Allyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Aryl-C₁-C₄-alkyl, oder Hetaryl-C₁-C₄-alkyl, das gegebenenfalls durch einen oder mehrere gleiche oder verschiedene der oben angegebenen Reste substituiert sein kann stehen,
- B: für Hydroxy oder tert.-Butoxy steht,
sowie deren optische Isomere und Racemate.

Ganz besonders bevorzugt werden erfindungsgemäß diejenigen Verbindungen der Formel (I) verwendet,
in welcher
- A: für Wasserstoff oder Benzyl steht,
- R¹, R⁴, R⁷ und R¹⁰: unabhängig voneinander für Methyl, Ethyl, Propyl und i-Propyl stehen,
- R², R⁵, R⁸ und R¹¹: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, C₂-C₈-Alkenyl, insbesondere Allyl, C₃-C₇-Cycloalkyl-C₁-C₄-alkyl, insbesondere Cyclohexylmethyl, Phenyl-C₁-C₄-alkyl, insbesondere Phenylmethyl stehen,
- R³, R⁶, R⁹ und R¹²: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, C₂-C₈-Alkenyl, insbesondere Vinyl, Allyl, C₃-C₇-Cycloalkyl-C₁-C₄-alkyl, insbesondere Cyclohexylmethyl, Aryl-C₁-C₄-alkyl, oder Hetaryl-C₁-C₄-alkyl, das gegebenenfalls durch einen oder mehrere gleiche oder verschiedene der oben angegebenen Reste substituiert sein kann stehen,
- B: für Hydroxy oder tert.-Butoxy steht,
sowie deren optische Isomere und Racemate.

Im einzelnen seien folgende Verbindungen der allgemeinen Formel (I) genannt, in welcher die Reste A und B die in der folgenden Tabelle angegebenen Bedeutungen haben: 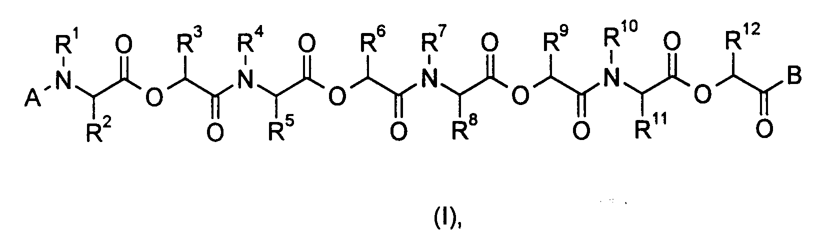 wobei
Bzl für Benzyl steht,
Bu für Butyl steht,
Et für Ethyl steht,
Me für Methyl steht,
Pr für Propyl steht.

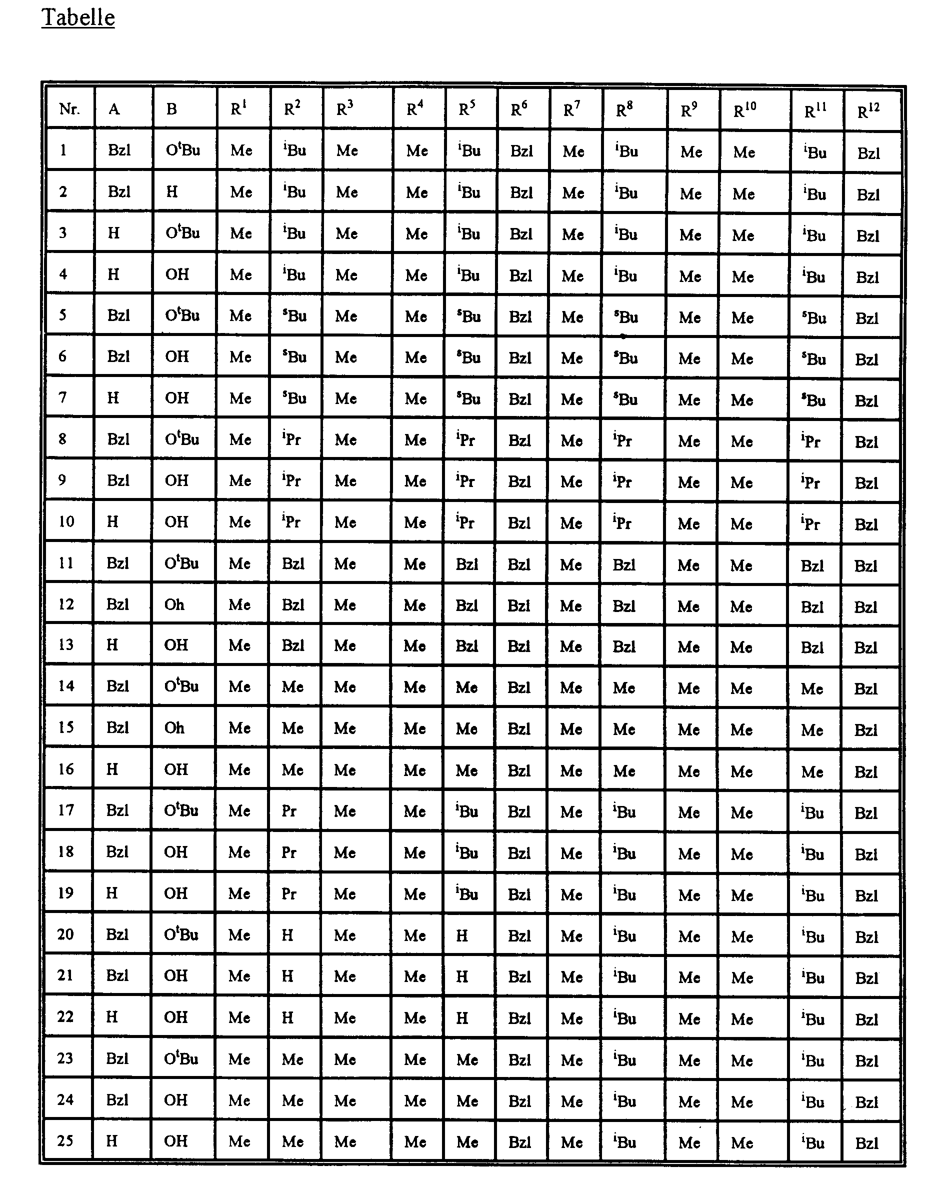

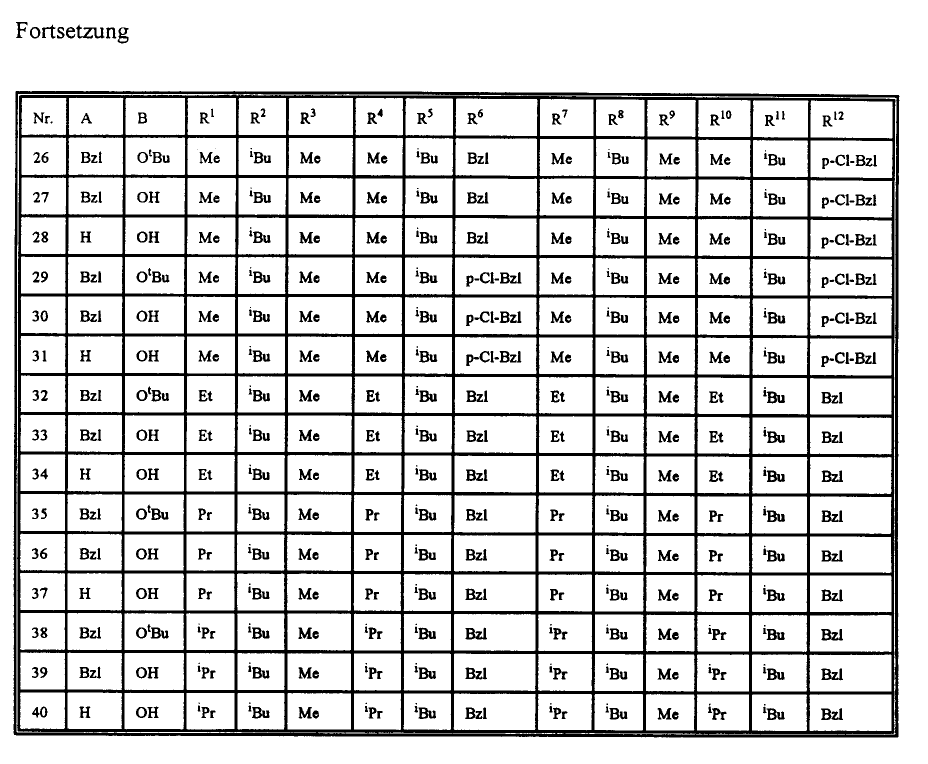

Die Verbindungen der allgemeinen Formel (I) können in optisch aktiven, stereoisomeren Formen oder als racemische Gemische vorliegen. Vorzugsweise werden jedoch die optisch aktiven Formen der Verbindungen der allgemeinen Formel (I) verwendet.

Die Herstellung der erfindungsgemäß zu verwendenden offenkettigen Octadepsipeptide der allgemeinen Formel (I) ist in vorgängigen, jedoch nicht vorveröffentlichten Patentanmeldungen beschrieben (vgl. Deutsche Patentanmeldungen P 4 317 457.4; P 4 317 432.9).

Man erhält die Verbindungen der Formel (I) 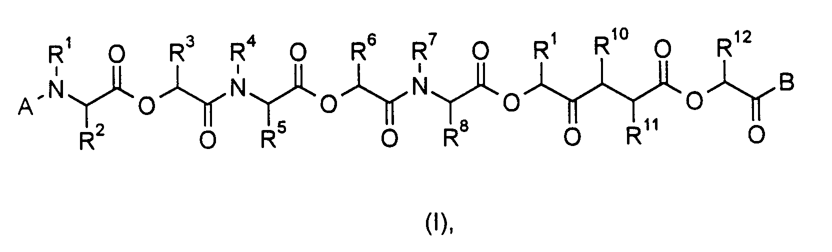 in welcher
A, R¹ bis R¹² und B die weiter oben angegebene Bedeutung besitzen, wenn man beispielsweise
Tetradepsipeptide der allgemeinen Formel (IIa) 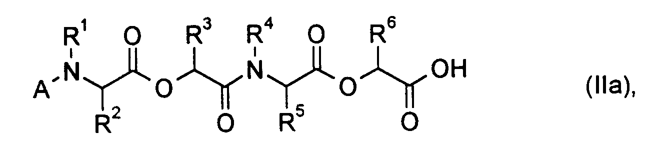 in welcher
A, R¹ bis R⁶ die oben angegebene Bedeutung haben,
mit Tetradepsipeptiden der allgemeinen Formel (IIIa) 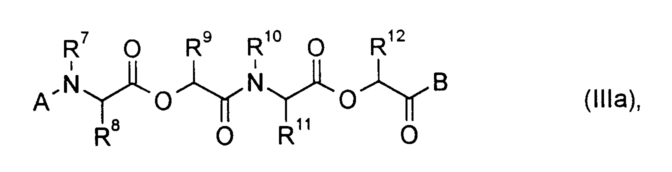 in welcher
R⁷ bis R¹² die weiter oben angegebene Bedeutung besitzen,
in Gegenwart geeigneter Kupplungsreagenzien, in Gegenwart eines basischen Reaktionshilfsmittels und in Gegenwart eines Verdünnungsmittels umsetzt.

Die offenkettigen Octadepsipeptide der Formel (I) können nach Klassischen Verfahren hergestellt werden, beispielweise demjenigen, wie es von H.-G. Lerchen und H. Kunz (Tetrahedron Lett. 26 (43) (1985) S. 5257-5260; 28 (17) (1987) S. 1873-1876) unter Ausnutzung der Veresterungsmethode nach B.F. Gisin (Helv. Chim. Acta 56 (1973) S. 1476) beschrieben ist.

Die als Ausgangsmaterialien für die Herstellung der Verbindungen der Formeln (IIa) und (IIIa) verwendeten N-Methyl-aminosäuren und 2-Halogen-carbonsäurederivate sind teilweise bekannt (vgl. z.B. N-Methyl-aminosäuren: R. Bowmann et al. J. Chem. Soc. (1950) S. 1346; J.R. McDermott et al. Can. J. Chem. 51 (1973) S. 1915; H. Wurzinger et al., Kontakte (Merck. Darmstadt) 3 (1987) S. 8; 2-Halogencarbonsäurederivate: S.M. Birnbaum et al. J. Amer. Chem. Soc. 76 (1954) S. 6054, C.S. Rondestvedt, Jr. et al. Org. Reactions 11 (1960) S. 189 [Review]) bzw. können nach den dort beschriebenen Verfahren erhalten werden.

Für die Kupplungsreaktion zur Darstellung der als Ausgangsverbindungen eingesetzten erfindungsgemäßen Tetradepsipeptide der Formeln (II), (III), finden alle Kupplungsreagenzien, die zur Herstellung einer Amidbindung geeignet sind (vgl. z.B. Houben-Weyl, Methoden der organischen Chemie, Band 15/2; Bodanszky et al., Peptide Synthesis 2nd ed. (Wiley & Sons, New York 1976) oder Gross, Meienhofer, The Peptides: Analysis synthesis, biology (Academic Press, New York 1979), Verwendung.

Die der Erfindung entsprechenden offenkettigen Octadepsipeptide der Formel (I) können somit nach der folgenden Reaktionssequenz erhalten werden, die die folgenden Stufen umfaßt:

### a) Synthese der Didepsipeptide der Formeln (IV) bis (VII):

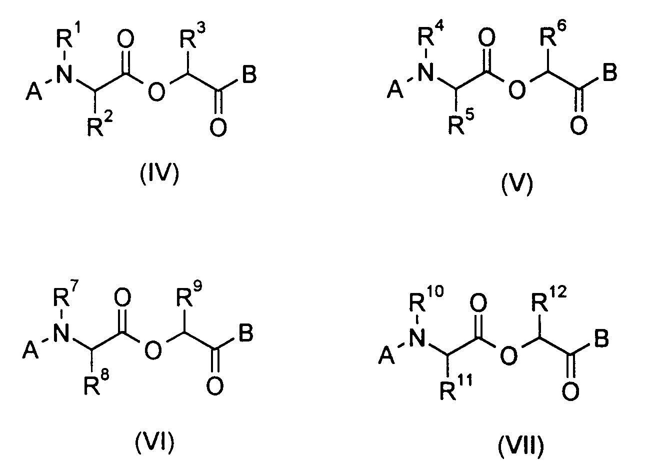

worin A eine N-terminale Schutzgruppe, wie z.B die Benzyl oder Benzyloxycarbonylgruppe und B eine C-terminale Schutzgruppe, wie z.B. die tert.-Butoxygruppe, darstellt.

Dies entspricht beispielsweise für Formel (IV) dem folgenden Reaktionsschema:

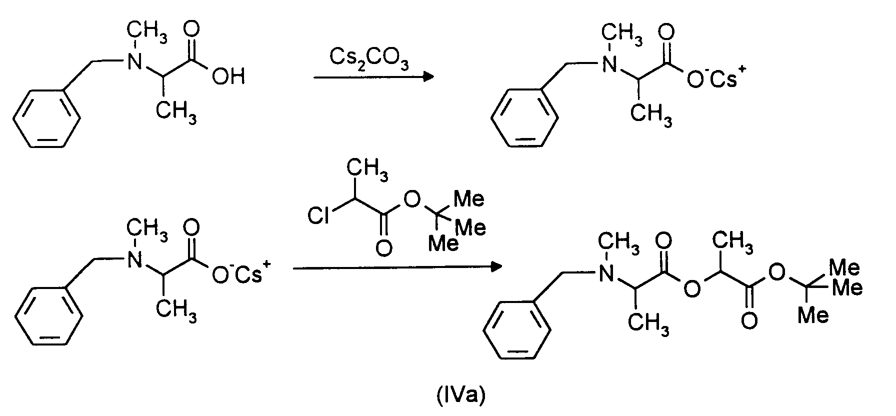

Die Herstellung der enantiomerenreinen Verbindungen der Formeln (IV), (V), (VI) und (VII) kann gegebenenfalls auch über die Trennung der Diastereomere nach üblichen Methoden wie beispielsweise Kristallisation, durch Säulenchromatographie oder durch Craig-Verteilung erfolgen. Welches das optimale Verfahren ist, muß von Fall zu Fall entschieden werden, manchmal ist es auch zweckmäßig, Kombinationen der einzelnen Verfahren zu benutzen.

Am Ende dieser Stufe kann entweder eine Entfernung der N-terminalen Schutzgruppe aus den Derivaten der Formel (V und VII) in an sich bekannter Weise durchgeführt werden, beispielsweise durch katalytische Hydrierung, zur Herstellung der Derivate der Formel (Va und VIIa) oder es kann in an sich bekannter Weise eine Abspaltung der C-terminalen Schutzgruppe aus den Derivaten der Formel (IV) und (VI), vorzugsweise durch Acidolyse, zur Darstellung der Derivate (IVb) und (VIb) erfolgen:

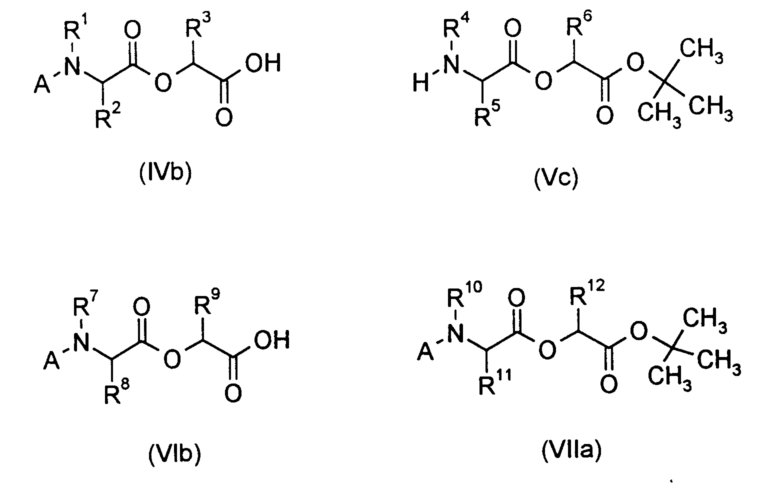

### b) Synthese der Tetradepsipeptide der Formel (II) und (III)

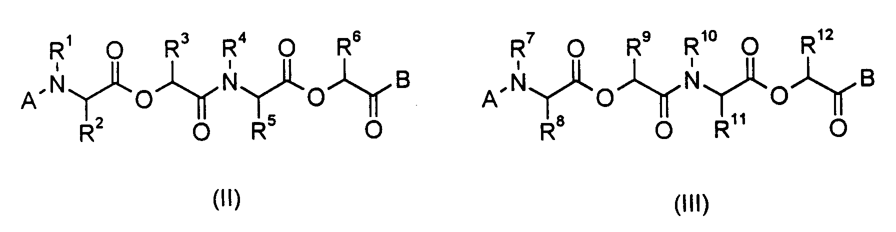

dies entspricht beispielsweise für Formel (IIb) dem nachfolgenden Reaktionsschema:

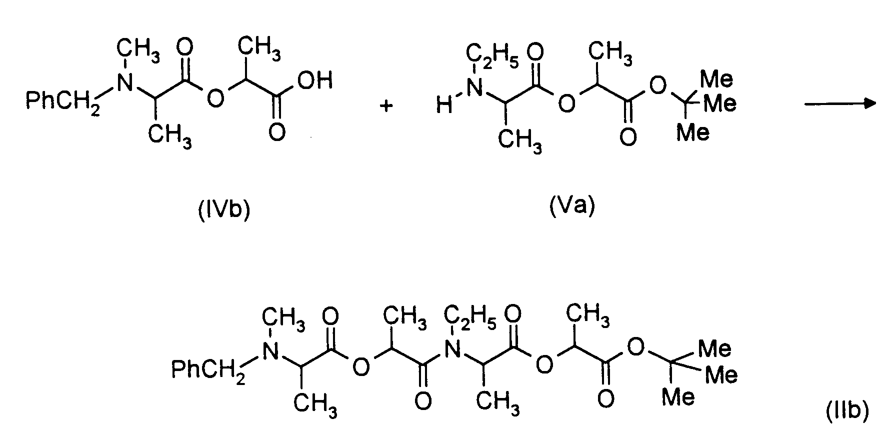

Man kann anschließend eine Entfernung der N-terminalen Schutzgruppe aus den Derivaten der Formel (IIb) durchführen, beispielsweise durch katalytische Hydrierung wie oben angegeben, zur Herstellung der Derivate der Formel

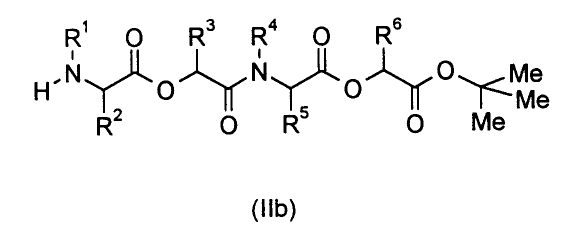

### c) Synthese der offenkettigen Octadepsipeptide der Formel

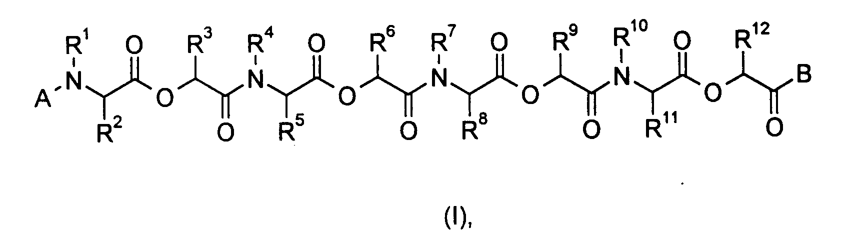

nach der folgenden Reaktionsgleichung:

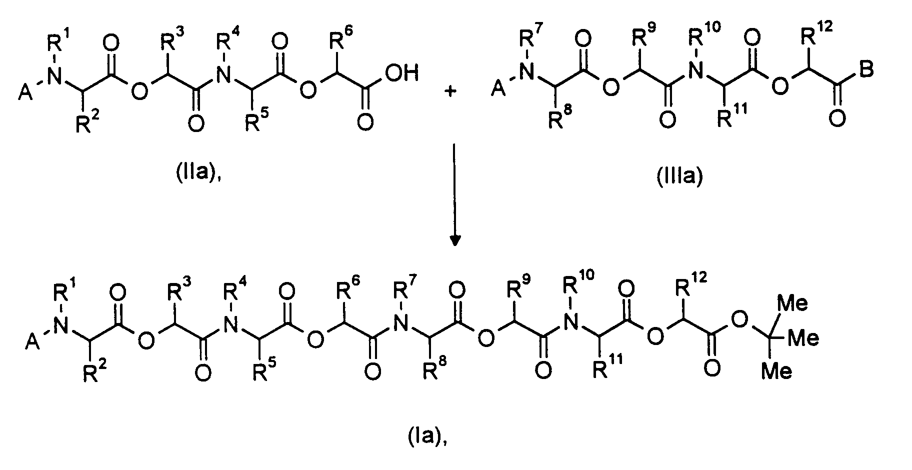

Man kann anschließend eine Entfernung der C-terminalen Schutzgruppe aus den Derivaten der Formel (Ia) in an sich bekannter Weise, beispielsweise durch Acidolyse, zur Herstellung der Derivate 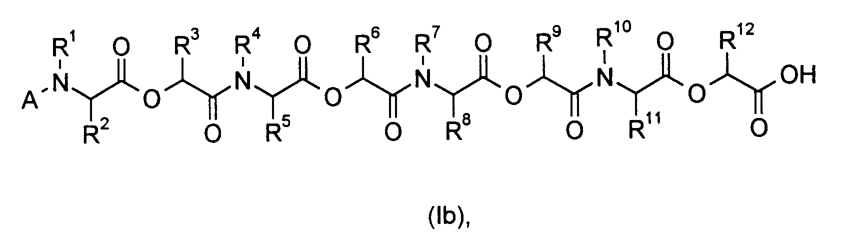 durchführen oder man deblockiert die Derivate der Formel (I) N-terminal in an sich bekannter Weise, beispielsweise durch katalytische Hydrierung wie oben angegeben, zur Herstellung der Derivate der Formel

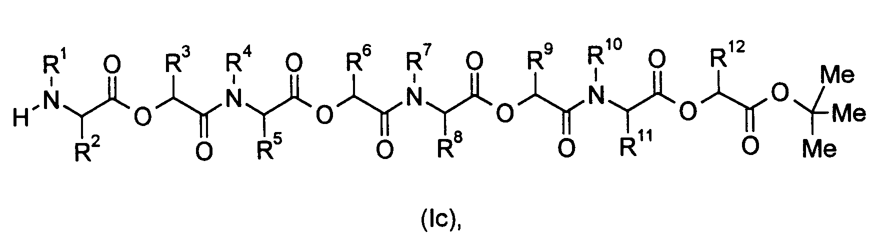

Am Ende dieser Stufen kann eine Entfernung der N-terminalen bzw. C-terminalen Schutzgruppe aus den Derivaten der Formel (Ib) bzw. (Ic) in an sich bekannter Weise durchgeführt werden, beispielsweise durch katalytische Hydrierung oder durch Acidolyse wie oben angegeben, zur Herstellung der Derivate der Formel

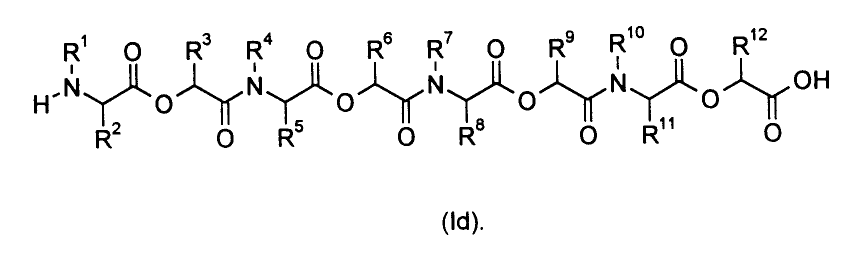

Die anfallenden Produkte lassen sich in üblicher Weise durch Umkristallisieren oder Säulenchromatographie reinigen (vgl. auch die Herstellungsbeispiele).

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp, Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen. Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmiffel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser. Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl / Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter ebentuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.
Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsaureester-monoethynolaminsalz;
Als weitere Hilfsstoffe seien genannt: Viskositätserhöhende und die Emulsion stabilisierende Stoffe wie Carboxymethylcellulose, Methylcellulose und andere Cellulose-und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside ganannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden, enthalten den Wirkstoff in Konzentrationen von 0,5 - 90 Gew.-%, bevorzugt von 5 - 50 Gew.-%.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

### Beispiel A

### In vivo Nematodentest

### Haemonchus contortus / Schaf

Experimentell mit Haemonchus contortus infizierte Schaft wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 1 | 10 |
| 2 | 10 |
| 3 | 10 |

### Herstellungsbeispiele

### Beispiel 1

### N-Benzyl-N-methyl-L-leucyl-D-lactyl-N-methyl-L-leucyl-D-3-phenyllactyl-N-methyl-L-leucyl-D-lactyl-N-methyl-L-leucyl-D-3-phenylmilchsäure-tert.-butyl-ester

H-L-MeLeu-D-Lac-L-MeLeu-D-PheLac-O^{t}Bu (1,38 g, 2,52 mmol) und Bzl-L-MeLeu-D-Lac-L-MeLeu-D-PheLac-OH (1,46 g, 2,52 mmol) wurden in Dichlormethan (15 ml) vorgelegt und die auf 0°C gekühlte Lösung mit Ethyldiisopropylamin (158,9 µl, 0,912 mmol) und BOP-Cl (111,5 mg, 0,438 mmol) versetzt. Es wurde 1 Stunde bei 0°C und 1,5 Stunden bei Raumtemperatur nachgerührt, dann mit Dichlormethan verdünnt, zweimal mit wenig Wasser gewaschen, über Natriumsulfat getrocknet und eingeengt. Flash-Chromatographie des Rückstandes an Kieselgel mit dem Laufmittel Cyclohexan-^{t}BuOMe=2:1 lieferte 2,18 g (79 %) Bzl-L-MeLeu-D-Lac-L-MeLeu-D-PheLac-L-MeLeu-D-Lac-L-MeLeu-D-PheLac-O^{t}Bu.

FAB-MS m/z (%): 1113 (M⁺+H, 100), 1023 (8), 909 (6), 710 (16).

### Beispiel 2

### N-Benzyl-N-propyl-L-leucyl-D-lactyl-N-ⁱpropyl-L-leucyl-D-3-phenyllactyl-N-ⁱpropyl-L-leucyl-D-lactyl-N-ⁱpropyl-L-leucyl-D-3-phenylmilchsäure-tert.-butylester

H-L-ⁱPrLeu-D-Lac-L-ⁱPrLeu-D-PheLac-O^{t}Bu (4,05 g, 6,69 mmol) und Bzl-L-ⁱPrLeu-D-Lac-L-ⁱPrLeu-D-PheLac-OH (4,48 g, 7,02 mmol) wurden in Dioxan (150 ml) vorgelegt und die auf 0°C gekühlte Lösung mit Ethyldiisopropylamin (2,16 g, 16,73 mmol) und BOP-Cl (2,04 g, 8,03 mmol) versetzt. Es wurde 30 Stunden unter Rückfluß gekocht, abgekühlt, eingeengt und in Dichlormethan aufgenommen. Es wurde zweimal mit wenig Wasser gewaschen, über Natriumsulfat getrocknet und eingeengt. Flash-Chromatographie des Rückstandes an Kieselgel mit dem Laufmittel ^{t}BuOMe-Cyclohexan = 1:5 lieferte 3,2 g (39 %) Bzl-L-ⁱPrLeu-D-Lac-L-ⁱPrLeu-D-PheLac-ⁱPrLeu-D-Lac-L-ⁱPrLeu-D-PheLac-O^{t}Bu.

FAB-MS m/z (%): 1225 (68, M⁺), 218 (100).

### Beispiel 3

H-L-MePhe-D-Lac-L-MePhe-D-PheLac-O^{t}Bu (1,96 g, 3,19 mmol) und Bzl-L-MePhe-D-Lac-L-MePhe-D-PheLac-OH (2,18 g, 3,35 mmol) wurden in Dichlormethan (20 ml) vorgelegt und die auf 0°C gekühlte Lösung mit Ethyldiisopropylamin (1,03 g, 7,97 mmol) versetzt. Es wurde 12 Stunden bei Raumtemperatur gerührt, zweimal mit wenig ges. NaHCO₃-Lösung gewaschen, über Natriumsulfat getrocknet und eingeengt. Flash-Chromatographie des Rückstandes an Kieselgel mit dem Laufmittel Cyclohexan-Ethylacetat = 4:1 lieferte 3,5 g (89 %) Bzl-L-MePhe-D-Lac-L-MePhe-D-PheLac-MePhe-D-Lac-L-MePhe-D-PheLac-O^{t}Bu.

FAB-MS m/z (%): 1249 (84, M⁺), 712 (43), 224 (100).

Analog können die in der nachstehenden Tabelle aufgeführten Verbindungen der allgemeinen Formel (I) als LDLDLDLD-Stereoisomere hergestellt werden.

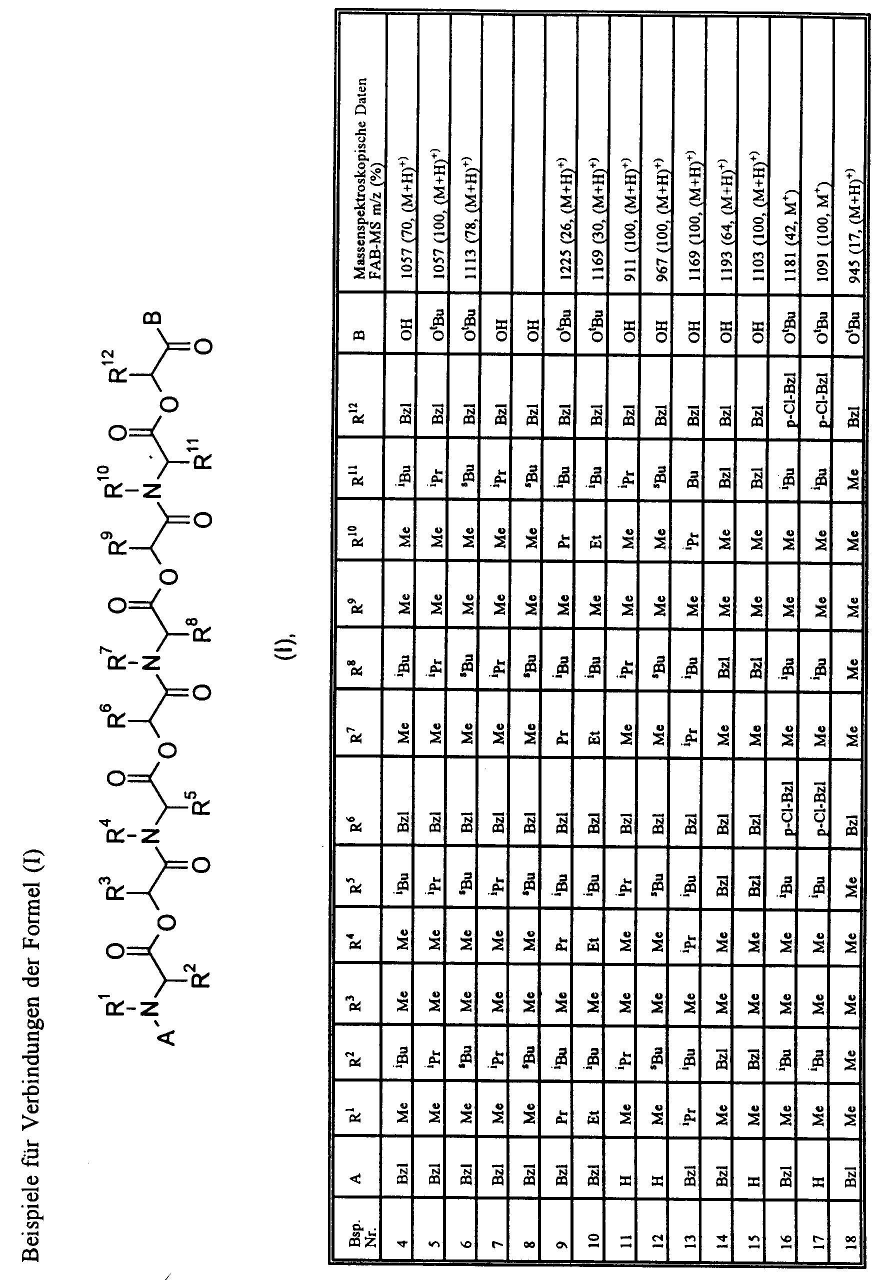

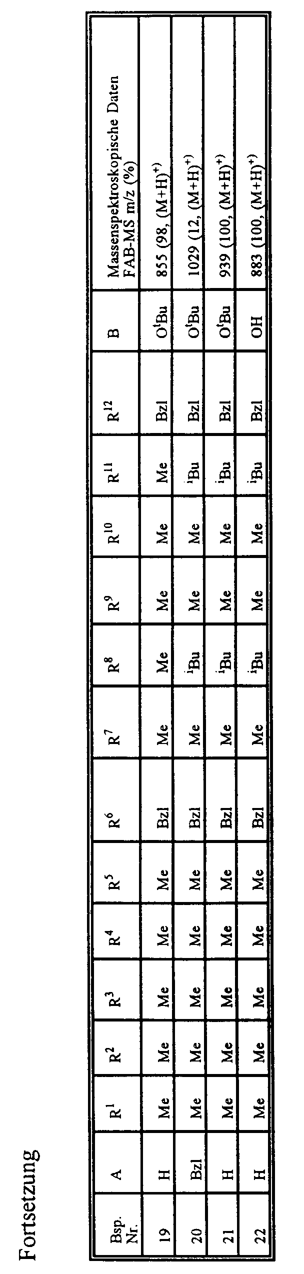

### Beispiele für die Herstellung der Ausgangsstoffe der Formeln (II) und (III)

### Beispiel II-1

### N-Benzyl-N-methyl-L-leucyl-D-lactyl-N-methyl-L-leucyl-D-3-phenylmilchsäure-tert.-butylester

Die Kupplungsreaktion erfolgt analog der Reaktionsvorschrift des Beispiels 1 unter Verwendung von:
- 8,0 g (22,9 mmol): L-MeLeu-D-Lac-L-MeLeu-D-PheLac-O^{t}Bu,
- 8,44 g (27,5 mmol): Bzl-L-MeLeu-D-Lac-L-MeLeu-D-PheLac-OH,
- 80 ml: Dichlormethan,
- 9,98 ml (57,3 mmol): Diisopropylethylamin,
- 7,59 g (29,8 mmol): Bis-(2-oxo-3-oxazolidinyl)-phosphoniumsäurechlorid.

Flash-Chromatographie des Rückstandes an Kieselgel mit dem Laufmittel Cyclohexan-Ethylacetat = 15:1 lieferte 11,6 g (80 %) Bzl-L-MeLeu-D-Lac-L-MeLeu-D-PheLac-L-MeLeu-D-PheLac-D-O^{t}Bu.

FAB-MS m/z (%): 639 (37, (M+H), 199 (100).

### Beispiel II-2

### N-Benzyl-N-methyl-L-leucyl-D-lactyl-N-methyl-L-leucyl-D-3-phenylmilchsäure

In eine auf 0°C gekühlte Lösung von Bzl-L-MeLeu-D-Lac-L-MeLeu-D-PheLac-O^{t}Bu (5,46 g, 8,56 mmol) in Dichlormethan (150 ml) wurde 2 Stunden HCl-Gas eingeleitet. Anschließend wurde auf Rt aufgewärmt und über Nacht gerührt. Nach Einengen und zweimaligem Aufnehmen in Dichlormethan wurde erneut einrotiert. Die in Wasser-Methanol = 1:1 (100 ml) gelöste Säure wurde 4 Stunden mit basischem Ionenaustauscher (15 g) verrührt. Nach Filtrieren, Einengen und Trocknen im Hochvakuum verbleiben 4,1 g (80 %) Bzl-L-MeLeu-D-Lac-L-MeLeu-D-PheLac-OH.
FAB-MS m/z (%): 583 (100 (M+H), 190 (64).

### Beispiel III-1

### N-Methyl-L-leucyl-D-lactyl-N-methyl-L-leucyl-D-3-phenylmilchsäure-tert.-butyl-ester

Bzl-L-MeLeu-D-Lac-L-MeLeu-D-PheLac-O^{t}Bu (6,08 g, 9,53 mmol) wurde in Ethanol (37 ml) aufgelöst, mit 0,6 g Pd(OH)₂/C (20 %) versetzt und bis zur Beendigung der Wasserstoffaufnahme bei Raumtemperatur hydriert. Nach Abfiltrieren des Katalysators und Einengen des Lösungsmittels ergab die säulenchromatographische Trennung des Rückstandes an Kieselgel mit dem Laufmittel ^{t}BuOMe-Cyclohexan-Ethanol = 1:1:0,05 387 g (74 %) L-MeLeu-D-Lac-L-MeLeu-D-PheLac-O^{t}Bu.

FAB-MS m/z (%): 549 (100, (M+H), 493 (58).

Analog können die in der folgenden Tabelle 3 aufgeführten Verbindungen als LDLD-Stereoisomere hergestellt werden.

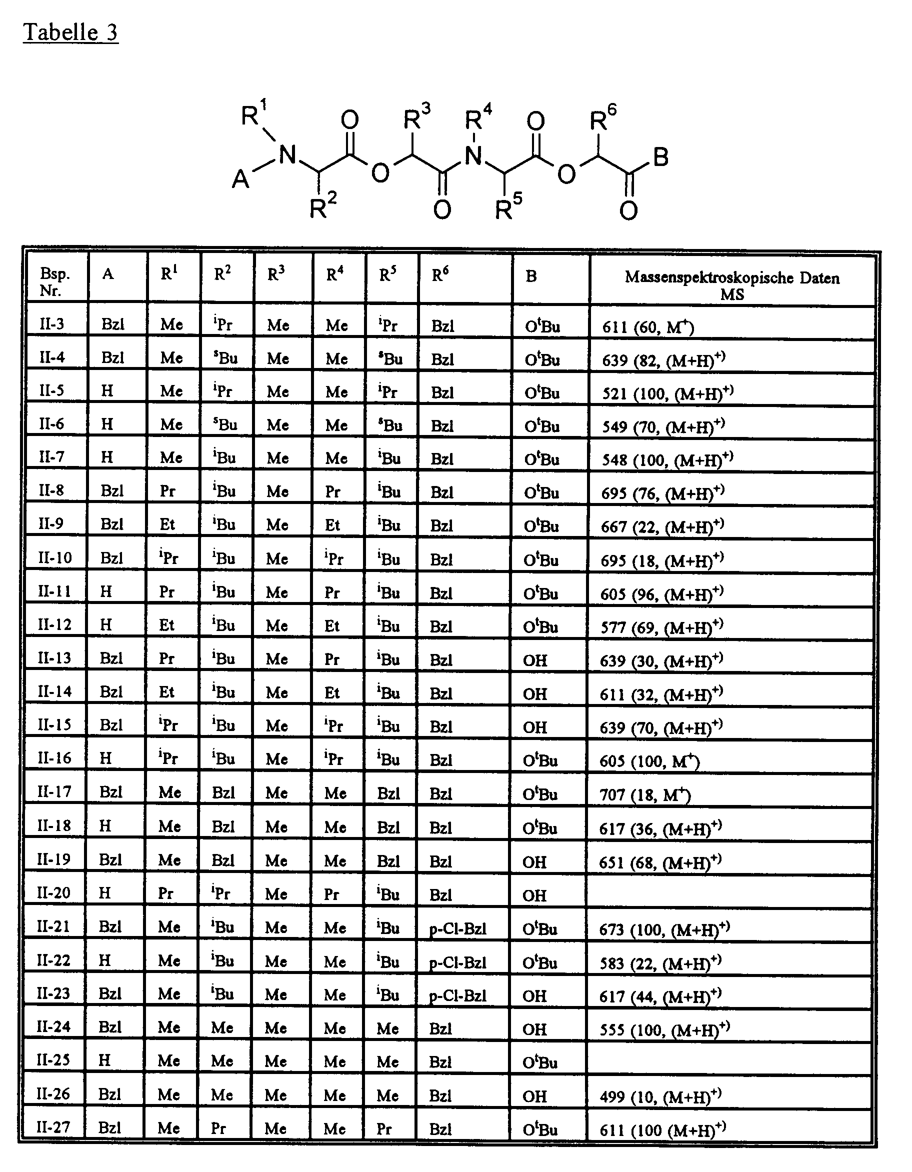

### Beispiele für die Herstellung der Ausgangsstoffe der Formel (IV) und (V)

### Beispiel (IV-1)

### N-Benzyl-N-methyl-L-leucyl-D-milchsäure-tert.-butylester

Das Caesiumsalz (77,7 g, 0,212 mol) von Bzl-L-MeLeu-OH wurde in Dimethylsulfoxid (530 ml) vorgelegt und bei Raumtemperatur mit 2-Chlor-propionsäure-tert.-butylester (34,76 g, 0,212 mol) versetzt. Es wurde 20 Stunden bei Raumtemperatur gerührt, in ges. Kochsalzlösung eingegossen und viermal mit Ethylacetat extrahiert. Die vereinigten organischen Extrakte wurde einmal mit wenig Wasser gewaschen, über Natriumsulfat getrocknet und eingeengt. Säulenchromatographie des Rückstandes an Kieselgel mit dem Laufmittel Cyclohexan-Ethylacetat = 60:1 ergab 63,5 g (82 %) Bzl-MeLeu-D-Lac-O^{t}Bu.

EI-MS m/z (%): 363 (M⁺,1), 190 (100).

### Beispiel (V-1)

### N-Benzyl-N-methyl-L-leucyl-D-3-phenylmilchsäure-tert.-butylester

Bzl-L-MeLeu-OH (50,0 g, 0,212 mol) wurde in Ethanol (1000 ml) und Wasser (100 ml) gelöst, mit einer 20%igen Caesiumcarbonatlösung (41,5 g, 0,12 mol) in Wasser versetzt und 5 Stunden bei Raumtemperatur gerührt. Anschließend wurde eingeengt, zweimal mit je 250 ml DMF destilliert und über Nacht bei 80°C im Hochvakuum getrocknet. Das Caesiumsalz (77,7 g, 0,212 mol) wurde in Dimethylsulfoxid (530 ml) vorgelegt, bei Raumtemperatur mit 2-Chlor-3-phenyl-propionsäure-tert.-butylester (51,0 g, 0,212 mol) versetzt und 20 Stunden bei Raumtemperatur gerührt. Die Lösung wurde in gesättigte Kochsalzlösung eingegossen, viermal mit Ethylacetat extrahiert, über Natriumsulfat getrocknet und eingeengt. Säulenchromatographie des Rückstandes an Kieselgel mit dem Laufmittel Cyclohexan-Ethylacetat = 100:1 lieferte 26,6 g (29%) sauberes Bzl-L-MeLeu-D-PheLac-O^{t}Bu sowie 48,9 g (52 %) einer mit Zimtsäure-tert.-butylester verunreinigten Mischfraktion.
EI-MS m/z (%): 363 (1), 190 (100).

Analog können die in der nachstehenden Tabelle aufgeführten Verbindungen als L-D-Stereoisomere hergestellt werden.

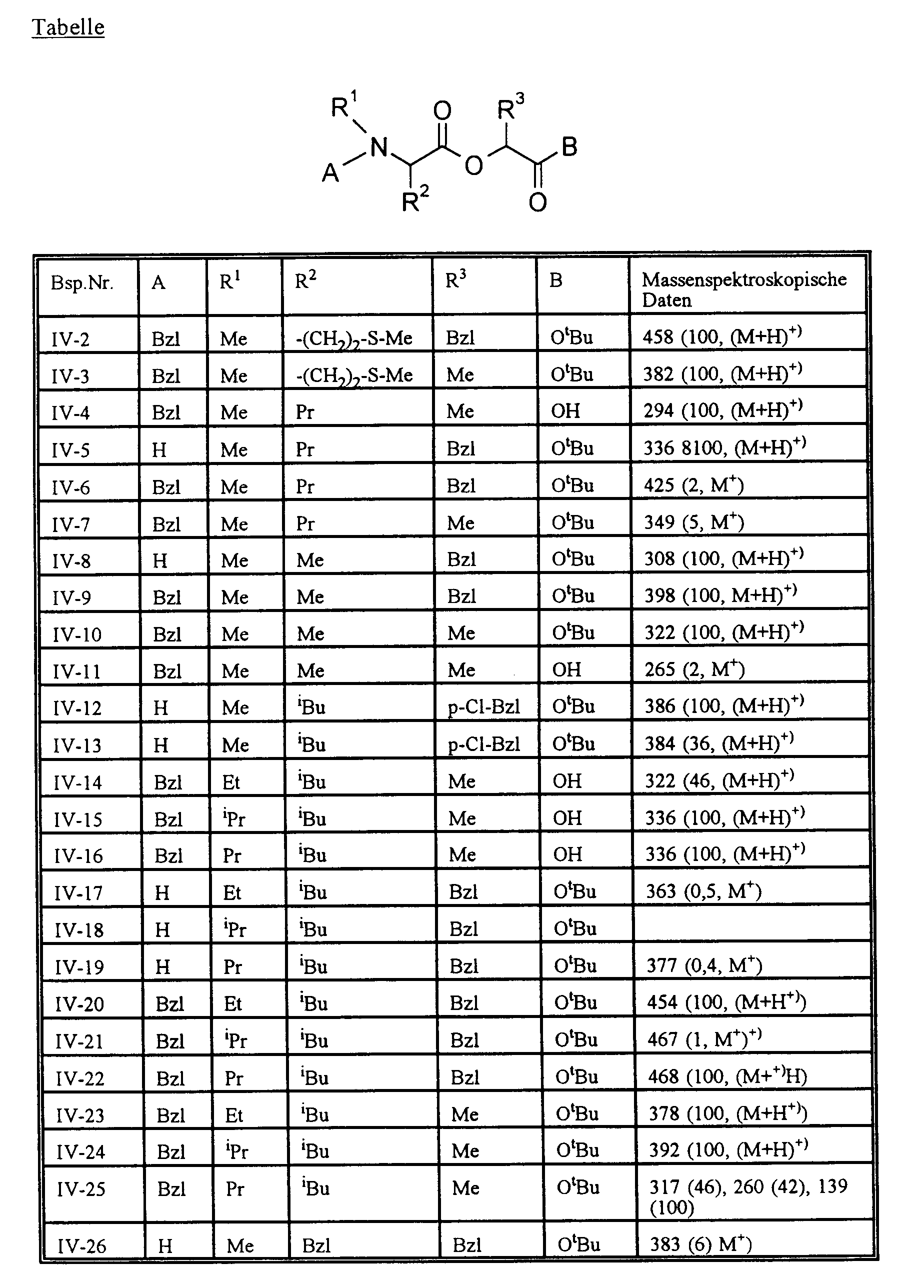

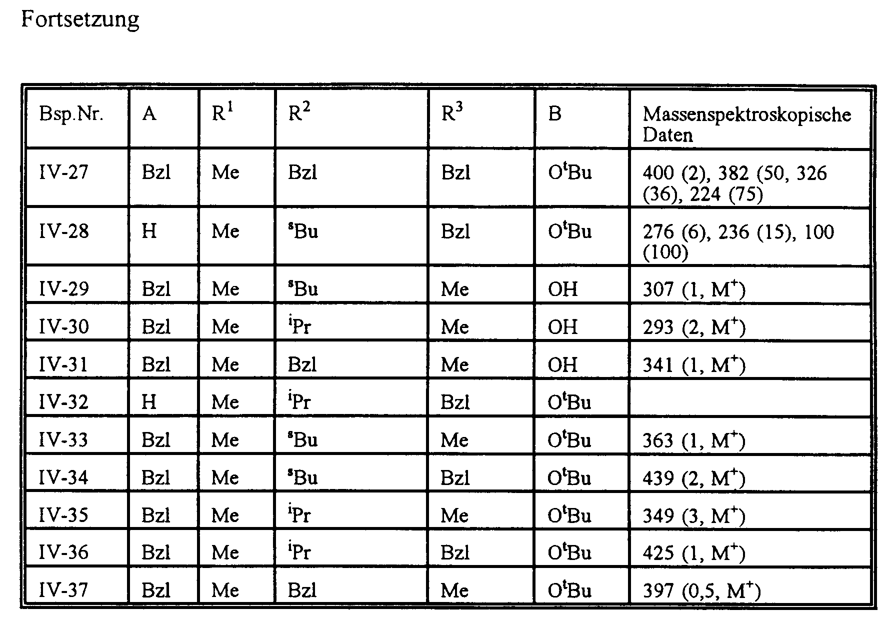

### Liste der verwendeten Abkürzungen:

- Leu: steht für Leucin
- Lac: steht für Milchsäure
- PheLac: steht für Phenylmilchsäure
- Bzl: steht für Benzyl
- Me: steht für Methyl
- BOP-Cl: steht für Bis-(2-oxo-3-oxcolidinyl)-phosphonylchlorid)
- ^{t}Bu: steht für tertiär-Butyl
- ⁱPr: steht für Isopropyl
- ^{s}Bu: steht für sekundär-Butyl
- Phe: für für Phenylalanin
- Et: steht für Ethyl
- Pr: steht für Propyl

## Patentansprüche

1. Verwendung der offenkettigen Octadepsipeptide der allgemeinen Formel (I) 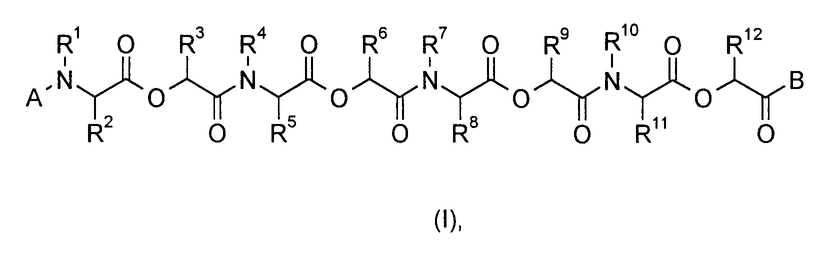 in welcher
A für Wasserstoff, Alkyl, Aralkyl oder einen Acylrest, insbesondere für einen Rest der Formel -CO-R¹⁵ steht, worin
R¹⁵ für geradkettiges, verzweigtes Alkyl, Alkenyl, Alkoxy, Aralkyl oder Arylalkoxy mit bis zu 6 Kohlenstoffatomen im Alkylteil steht,
R¹, R⁴, R⁷ und R¹⁰ unabhängig voneinander für C₁-C₈-Alkyl, C₃-C₆-Cycloalkyl oder Aralkyl stehen,
R², R⁵, R⁸ und R¹¹ unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie gegebenenfalls substituiertes Arylalkyl, wobei als Substituenten genannt seien Halogen, Hydroxy, Alkyl, Alkoxy stehen,
R³, R⁶, R⁹ und R¹² unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Alkylthioalkyl, Alkylsulfinylalkyl, Alkysulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Alkoxycarbonylaminoalkyl, Alkenyl, Cycloalkyl, Cycloalkylalkyl sowie gegebenenfalls substituiertes Aryl, Hetaryl, Arylalkyl oder Hetarylalkyl, wobei als Substituenten genannt seien Halogen, Hydroxy, Alkyl, Alkoxy, Nitro oder 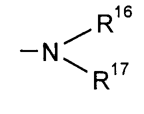 stehen, wobei jeder Rest Wasserstoff, Alkoxy oder beide Reste zusammen einen 5-, 6- oder 7-gliedrigen Ring bilden können, der gegebenenfalls durch O, S oder N unterbrochen ist und der gegebenenfalls mit C₁₋₄-alkyl substituiert ist.
B für Hydroxy, Alkoxy mit bis zu 4 Kohlenstoffatomen oder den Rest NR¹³R¹⁴ steht, wobei
R¹³ und R¹⁴ für Wasserstoff, Alkyl, Aralkyl oder Aryl stehen,
sowie deren optische Isomere und Racemate,
zur Herstellung von Mitteln zur Bekämpfung von Endoparasiten in der Medizin und Tiermedizin.

2. Endoparasitizide Mittel, gekennzeichnet durch einen Gehalt an mindestens einer Verbindung der Formel (I) gemäß Anspruch 1.

3. Verfahren zur Herstellung von endoparasitiziden Mitteln, dadurch gekennzeichnet, daß man Verbindungen der Formel (I) gemäß Anspruch 1 mit Streckmitteln und/oder oberflächenaktiven Mitteln vermischt.

4. Verwendung von offenkettigen Octadepsipeptiden der Formel (I) gemäß Anspruch 1,
in welcher
A für Wasserstoff, C₁-C₄-Alkyl oder Benzyl steht,
oder für eine Gruppe der Formel -CO-R¹⁵ steht,
worin
R¹⁵ für geradkettiges oder verzweigtes C₁-C₄-Alkyl, C₂-C₄-Alkenyl, C₁-C₄-Alkoxy oder Phenylalkoxy mit bis zu 6 Kohlenstoffatomen im Alkylteil steht,
R¹, R⁴, R⁷ und R¹⁰ unabhängig voneinander für C₁-C₄-Alkyl, C₃-C₆-Cycloalkyl, Phenyl-C₁₋₄-alkyl stehen,
R², R⁵, R⁸ und R¹¹ unabhängig voneinander für Wasserstoff, geradkettiges oder verzweigtes C₁-C₈-Alkyl, Hydroxy-C₁-C₆-alkyl, C₁-C₄-Alkanoyloxy-C₁-C₆-alkyl, C₁-C₄-Alkoxy-C₁-C₆-alkyl, Aryl-C₁-C₄-alkyloxy-C₁-C₆-alkyl, Mercapto-C₁-C₆-alkyl, C₁-C₄-Alkylthio-C₁-C₆-alkyl, C₁-C₄-Alkylsulfinyl-C₁-C₆-alkyl, C₁-C₄-Alkylsulfonyl-C₁-C₆-alkyl, Carboxy-C₁-C₆-alkyl, C₁-C₄-Alkoxycarbonyl-C₁-C₆-alkyl, C₁₋C₄-Arylalkoxycarbonyl-C₁-C₆-alkyl, Carbamoyl-C₁-C₆-alkyl, Amino-C₁-C₆-alkyl, C₁-C₄-Alkylamino-C₁-C₆-alkyl, C₁-C₄-Dialkylamino-C₁-C₆-alkyl, Guanido-C₁-C₆-alkyl, C₁-C₄-Alkoxycarbonylamino-C₁-C₆-alkyl, C₂-C₈-Alkenyl, C₃-C₇-Cycloalkyl, C₃-C₇-Cycloalkyl-C₁-C₄-alkyl, Phenyl-C₁-C₄-alkyl, das gegebenenfalls durch Reste aus der Reihe Halogen, C₁-C₄-Alkoxy, C₁-C₄-Alkyl, substituiert sein kann stehen,
R³, R⁶, R⁹ und R¹² unabhängig voneinander für Wasserstoff, geradkettiges oder verzweigtes C₁-C₈-Alkyl, Hydroxy-C₁-C₆-alkyl, C₁-C₄-Alkanoyloxy-C₁-C₆-alkyl, C₁-C₄-Alkoxy-C₁-C₆-alkyl, Aryl-C₁-C₄-alkyloxy-C₁-C₆-alkyl, Mercapto-C₁-C₆-alkyl, C₁-C₄-Alkylthio-C₁-C₆-alkyl, C₁-C₄-Alkylsulfinyl-C₁-C₆-alkyl, C₁-C₄-Alkylsulfonyl-C₁-C₆-alkyl, Carboxy-C₁-C₆-alkyl, C₁-C₄-Alkoxycarbonyl-C₁-C₆-alkyl, C₁-C₄-Arylalkoxycarbonyl-C₁-C₆-alkyl, Carbamoyl-C₁-C₆-alkyl, Amino-C₁-C₆-alkyl, C₁-C₄-Alkylamino-C₁-C₆-alkyl, C₁-C₄-Dialkylamino-C₁-C₆-alkyl, Guanido-C₁-C₆-alkyl, C₁-C₄-Alkoxycarbonylamino-C₁-C₆-alkyl, C₂-C₈-Alkenyl, C₃-C₇-Cycloalkyl, C₃-C₇-Cycloalkyl-C₁-C₄-alkyl, Aryl-C₁-C₄-alkyl oder Hetaryl-C₁-C₄-alkyl stehen, die gegebenenfalls durch Reste substituiert sein können aus der Reihe Halogen, Hydroxy, C₁-C₄-Alkoxy, C₁-C₄-Alkyl, Nitro oder eine Aminogruppe der Formel 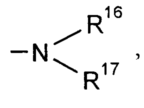 in der jeder Rest R¹⁶ oder R¹⁷ für Wasserstoff, C₁₋₄-Alkyl, C₁₋₄-Alkoxy oder beide Reste zusammen einen 5-, 6- oder 7-gliedrigen Ring bilden können, der gegebenenfalls durch O, S oder N unterbrochen ist und der gegebenenfalls mit C₁-C₄-Alkyl substituiert ist,
B für Hydroxy, tert.-Butoxy oder den Rest NR¹³R¹⁴ steht, wobei
R¹³ und R¹⁴ unabhängig voneinander für Wasserstoff, geradkettiges oder verzweigtes C₁₋₄-Alkyl, Benzyl, Cyclopropyl, Cyclohexyl, Phenyl stehen
sowie deren optische Isomere und Racemate.

5. Verwendung von offenkettigen Octadepsipeptiden der Formel (I) gemäß Anspruch 1
in welcher
A für Wasserstoff, Benzyl oder eine Gruppe COR¹⁵ steht,
R¹⁵ für geradkettiges oder verzweigtes Alkoxy oder Arylalkoxy mit bis zu 6 Kohlenstoffatomen im Alkylteil steht,
R¹, R⁴, R⁷ und R¹⁰ unabhängig voneinander für C₁₋₄-Alkyl, C₃₋₆-Cycloalkyl und Benzyl stehen,
R², R⁵, R⁸ und R¹¹ unabhängig voneinander für Wasserstoff, geradkettiges oder verzweigtes C₁-C₈-Alkyl, C₁-C₄-Alkanoyloxy-C₁-C₆-alkyl, C₁-C₄-Alkoxy-C₁-C₆-alkyl, Aryl-C₁-C₄-alkyloxy-C₁-C₆-alkyl, C₁-C₄-Alkoxycarbonylamino-C₁-C₆-alkyl, C₂-C₈-Alkenyl, C₃-C₇-Cycloalkyl, C₃-C₇-Cycloalkyl-C₁-C₄-alkyl, Phenyl-C₁-C₄-alkyl, das gegebenenfalls durch einen oder mehrere gleiche oder verschiedene Reste aus der Reihe Halogen, Methoxy, Ethoxy, Methyl substituiert sein kann stehen,
R³, R⁶, R⁹ und R¹² unabhängig voneinander für Wasserstoff, geradkettiges oder verzweigtes C₁-C₈-Alkyl, C₁-C₄-Alkanoyloxy-C₁-C₆-alkyl, C₁-C₄-Alkoxy-C₁-C₆-alkyl, Aryl-C₁-C₄-alkyloxy-C₁-C₆-alkyl, C₁-C₄-Alkoxycarbonylamino-C₁-C₆-alkyl, C₂-C₈-Alkenyl, C₃-C₇-Cycloalkyl, C₃-C₇-Cycloalkyl-C₁-C₄-alkyl, Aryl-C₁-C₄-alkyl, oder Hetaryl-C₁-C₄-alkyl, das gegebenenfalls durch einen oder mehrere gleiche oder verschiedene Reste aus der Reihe Halogen, Methoxy, Ethoxy, Nitro oder eine Aminogruppe der Formel -NR¹⁶R¹⁷ wobei R¹⁶ und R¹⁷ die in Anspruch 4 angegebene Bedeutung haben substituiert sein kann stehen,
B für Hydroxy oder tert.-Butoxy steht,
sowie deren optische Isomere und Racemate.

6. Verwendung von offenkettigen Octadepsipentiden der Formel (I) gemäß Anspruch 1
in welcher
A für Wasserstoff oder Benzyl steht,
R¹, R⁴, R⁷ und R¹⁰ unabhängig voneinander für Methyl, Ethyl, Propyl und i-Propyl stehen,
R², R⁵, R⁸ und R¹¹ unabhängig voneinander für Wasserstoff, Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, Allyl, Cyclohexylmethyl, Phenylmethyl stehen,
R³, R⁶, R⁹ und R¹² unabhängig voneinander für Wasserstoff, Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, Vinyl, Allyl, Cyclohexylmethyl, Aryl-C₁-C₄-alkyl, oder Hetaryl-C₁-C₄-alkyl, das gegebenenfalls durch einen oder mehrere gleiche oder verschiedene der in Anspruch 4 angegebenen Reste substituiert sein kann stehen,
B für Hydroxy oder tert.-Butoxy steht,
sowie deren optische Isomere und Racemate.

## Claims

1. Use of the open-chain octadepsipeptides of the general formula (I) 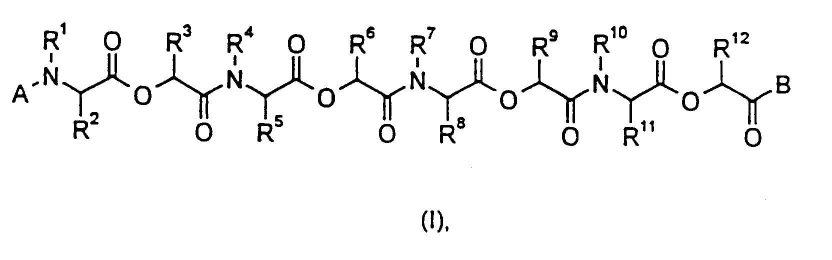 in which
A represents hydrogen, alkyl, aralkyl or an acyl radical, in particular a radical of the formula -CO-R¹⁵ in which
R¹⁵ represents straight-chain or branched alkyl, alkenyl, alkoxy, aralkyl or arylalkoxy having up to 6 carbon atoms in the alkyl moiety,
R¹, R⁴, R⁷ and R¹⁰ independently of one another represent C₁-C₈-alkyl, C₃-C₆-cycloalkyl or aralkyl,
R², R⁵, R⁸ and R¹¹ independently of one another represent hydrogen, straight-chain or branched alkyl having up to 8 carbon atoms, hydroxyalkyl, alkanoyloxyalkyl, alkoxyalkyl, aryloxyalkyl, mercaptoalkyl, alkylthioalkyl, alkylsulphinylalkyl, alkylsulphonylalkyl, carboxyalkyl, alkoxycarbonylalkyl, arylalkoxycarbonylalkyl, carbamoylalkyl, aminoalkyl, alkylaminoalkyl, dialkylaminoalkyl, guanidinoalkyl, which can optionally be substituted by one or two benzyloxycarbonyl radicals or by one, two, three or four alkyl radicals, or represent alkoxycarbonylaminoalkyl, 9-fluorenylmethoxycarbonyl(Fmoc)aminoalkyl, alkenyl, cycloalkyl, cycloalkylalkyl and optionally substituted arylalkyl, substituents which may be mentioned being halogen, hydroxyl, alkyl and alkoxy,
R³, R⁶, R⁹ and R¹² independently of one another represent hydrogen, straight-chain or branched alkyl having up to 8 carbon atoms, hydroxyalkyl, alkanoyloxyalkyl, alkoxyalkyl, aryloxyalkyl, alkylthioalkyl, alkylsulphinylalkyl, alkylsulphonylalkyl, carboxyalkyl, alkoxycarbonylalkyl, arylalkoxycarbonylalkyl, carbamoylalkyl, aminoalkyl, alkylaminoalkyl, dialkylaminoalkyl, alkoxycarbonylaminoalkyl, alkenyl, cycloalkyl, cycloalkylalkyl and optionally substituted aryl, hetaryl, arylalkyl or hetarylalkyl, substituents which may be mentioned being halogen, hydroxyl, alkyl, alkoxy, nitro or 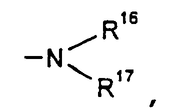 it being possible for each radical to be hydrogen or alkoxy or for both radicals together to form a 5-, 6- or 7-membered ring which is optionally interrupted by O, S or N and which is optionally substituted by C₁₋₄-alkyl,
B represents hydroxyl, alkoxy having up to 4 carbon atoms or the radical NR¹³R¹⁴ in which
R¹³ and R¹⁴ represent hydrogen, alkyl, aralkyl or aryl,
and their optical isomers and racemates,
for the preparation of compositions for combating endoparasites in medicine and veterinary medicine.

2. Endoparasiticidal compositions, characterized in that they contain at least one compound of the formula (I) according to Claim 1.

3. Process for the preparation of endoparasiticidal compositions, characterized in that compounds of the formula (I) according to Claim 1 are mixed with extenders and/or surfactants.

4. Use of open-chain octadepsipeptides of the formula (I) according to Claim 1,
in which
A represents hydrogen, C₁-C₄-alkyl or benzyl
or represents a group of the formula -CO-R¹⁵
in which
R¹⁵ represents straight-chain or branched C₁-C₄-alkyl, C₂-C₄-alkenyl, C₁-C₄-alkoxy or phenylalkoxy having up to 6 carbon atoms in the alkyl moiety,
R¹, R⁴, R⁷ and R¹⁰, independently of one another represent C₁-C₄-alkyl, C₃-C₆-cycloalkyl or phenyl-C₁₋₄-alkyl,
R², R⁵, R⁸ and R¹¹, independently of one another represent hydrogen, straight-chain or branched C₁-C₈-alkyl, hydroxy-C₁-C₆-alkyl, C₁-C₄-alkanoyloxy-C₁-C₆-alkyl, C₁-C₄-alkoxy-C₁-C₆-alkyl, aryl-C₁-C₄-alkyloxy-C₁-C₆-alkyl, mercapto-C₁-C₆-alkyl, C₁-C₄-alkylthio-C₁-C₆-alkyl, C₁-C₄-alkylsulphinyl-C₁-C₆-alkyl, C₁-C₄-alkylsulphonyl-C₁-C₆-alkyl, carboxy- C₁-C₆-alkyl, C₁-C₄-alkoxycarbonyl-C₁-C₆-alkyl, C₁-C₄-arylalkoxycarbonyl-C₁-C₆-alkyl, carbamoyl-C₁-C₆-alkyl, amino-C₁-C₆-alkyl, C₁-C₄-alkylamino-C₁-C₆-alryl, C₁-C₄-dialkylamino-C₁-C₆-alkyl, guanido-C₁-C₆-alkyl, C₁-C₄-alkoxycarbonylamino-C₁-C₆-alkyl, C₂-C₈-alkenyl, C₃-C₇-cycloalkyl, C₃-C₇-cycloalkyl-C₁-C₄-alkyl or phenylC₁-C₄-alkyl which can optionally be substituted by radicals from the series consisting of halogen, C₁-C₄-alkoxyl and C₁-C₄-alkyl,
R³, R⁶, R⁹ and R¹² independently of one another represent hydrogen, straight-chain or branched C₁-C₈-alkyl, hydroxy-C₁-C₆-alkyl, C₁-C₄-alkanoyloxy-C₁-C₆-alkyl, C₁-C₄-alkoxy-C₁-C₆-alkyl, aryl-C₁-C₄-alkyloxy- C₁-C₆-alkyl, mercapto-C₁-C₆-alkyl, C₁-C₄-alkylthio-C₁-C₆-alkyl, C₁-C₄-alkylsulphinyl-C₁-C₆-alkyl, C₁-C₄-alkylsulphonyl-C₁-C₆-alkyl, carboxyC₁-C₆-alkyl, C₁-C₄-alkoxycarbonyl-C₁-C₆-alkyl, C₁-C₄-arylalkoxycarbonyl-C₁-C₆-alkyl, carbamoyl-C₁-C₆-alkyl, amino-C₁-C₆-alkyl, C₁-C₄-alkylamino-C₁-C₆-alkyl, C₁-C₄-dialkylamino-C₁-C₆-alkyl, guanido-C₁-C₆-alkyl, C₁-C₄-alkoxycarbonylamino-C₁-C₆-alkyl, C₂-C₈-alkenyl, C₃-C₇-cycloalkyl, C₃-C₇-cycloalkyl-C₁-C₄-alkyl, aryl-C₁-C₄-alryl or hetaryl-C₁-C₄-alkyl, all of which can optionally be substituted by radicals selected from the series consisting of halogen, hydroxyl, C₁-C₄-alkoxy, C₁-C₄-alkylyl, nitro or an amino group of the formula 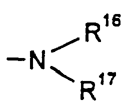 in which each radical R¹⁶ or R¹⁷ is hydrogen, C₁₋₄-alkyl or C₁₋₄-alkoxy or both radicals together can form a 5-, 6- or 7-membered ring which is optionally interrupted by O, S or N and which is optionally substituted by C₁-C₄- alkyl, and
B represents hydroxyl, tert-butoxy or the radical NR¹³R¹⁴ where
R¹³ and R¹⁴ independently of one another represent hydrogen, straight-chain or branched C₁-₄-alkyl, benzyl, cyclopropyl, cyclohexyl or phenyl,
and their optical isomers and racemates.

5. Use of open-chain octadepsipeptides of the formula (I) according to Claim 1
in which
A represents hydrogen, benzyl or a group COR¹⁵,
R¹⁵ represents straight-chain or branched alkoxy or arylalkoxy having up to 6 carbon atoms in the alkyl moiety,
R¹, R⁴, R⁷ and R¹⁰ independently of one another represent C₁₋₄-alkyl, C₃₋₆-cycloalkyl and benzyl,
R², R⁵, R⁸ and R¹¹, independently of one another represent hydrogen, straight-chain or branched C₁-C₈-alkyl, C₁-C₄-alkanoyloxy-C₁-C₆-alkyl, C₁-C₄-alkoxy-C₁-C₆-alkyl, aryl-C₁-C₄-alkyloxy-C₁-C₆-alkyl, C₁-C₄-alkoxycarbonylamino-C₁-C₆-alkyl, C₂-C₈-alkenyl, C₃-C₇-cycloalkyl, C₃-C₇-cycloalkyl-C₁-C₄-alkyl or phenyl-C₁-C₄-alkyl which can optionally be substituted by one or more identical or different radicals from the series consisting of halogen, methoxy, ethoxy and methyl,
R³, R⁶, R⁹ and R¹² independently of one another represent hydrogen, straight-chain or branched C₁-C₈-alkyl, C₁-C₄-alkanoyloxy-C₁-C₆-alkyl, C₁-C₄-alkoxy-C₁-C₆-alkyl, aryl-C₁-C₄-alkyloxy-C₁-C₆-alkyl, C₁-C₄-alkoxycarbonylamino-C₁-C₆-alkyl, C₂-C₈-alkenyl, C₃-C₇-cycloalkyl, C₃-C₇-cycloalkyl-C₁-C₄-alkyl, aryl-C₁-C₄-alkyl or hetaryl-C₁-C₄-alkyl which can optionally be substituted by one or more identical or different radicals from the series consisting of halogen, methoxy, ethoxy, nitro or an amino group of the formula -NR¹⁶R¹⁷ where R¹⁶ and R¹⁷ are as defined in Claim 4,
B represents hydroxyl or tert-butoxy,
and their optical isomers and racemates.

6. Use of open-chain octadepsipeptides of the formula (I) according to Claim 1
in which
A represents hydrogen or benzyl,
R¹, R⁴, R⁷ and R¹⁰ independently of one another represent methyl, ethyl, propyl and i-propyl,
R², R⁵, R⁸ and R¹¹, independently of one another represent hydrogen, methyl, ethyl, propyl, isopropyl, butyl, isobutyl, sec-butyl, pentyl, isopentyl, sec-pentyl, hexyl, isohexyl, sec-hexyl, heptyl, isoheptyl, sec-heptyl, octyl, isooctyl, sec-octyl, allyl, cyclohexylmethyl and phenylmethyl,
R³, R⁶, R⁹ and R¹² independently of one another represent hydrogen, methyl, ethyl, propyl, isopropyl, butyl, isobutyl, secbutyl, pentyl, isopentyl, sec-pentyl, hexyl, isohexyl, sec-hexyl, heptyl, isoheptyl, sec-heptyl, octyl, isooctyl, sec-octyl, vinyl, allyl, cyclohexylmethyl, aryl-C₁-C₄-alkyl or hetaryl-C₁-C₄-alkyl which can optionally be substituted by one or more identical or different radicals from amongst those given in Claim 4, and
B represents hydroxyl or tert-butoxy
and their optical isomers and racemates.

## Revendications

1. Utilisation des octadepsipeptides à chaîne ouverte de formule générale (I) 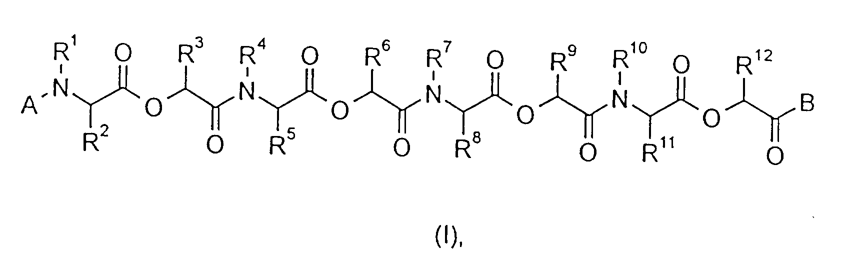 dans laquelle
A représente l'hydrogène, un groupe alkyle, aralkyle ou un reste acyle, notamment un reste de formule -CO-R¹⁵, dans laquelle
R¹⁵ est un groupe alkyle, alcényle, alkoxy, aralkyle ou arylalkoxy linéaire ou ramifié ayant jusqu'à 6 atomes de carbone dans la partie alkyle,
R¹, R⁴, R⁷ et R¹⁰ représentent, indépendamment les uns des autres, un groupe alkyle en C₁ à C₈, cycloalkyle en C₃ à C₆ ou aralkyle,
R², R⁵, R⁸ et R¹¹ représentent, indépendamment les uns des autres, l'hydrogène, un groupe alkyle linéaire ou ramifié ayant jusqu'à 8 atomes de carbone, hydroxyalkyle, alcanoyloxyalkyle, alkoxyalkyle, aryloxyalkyle, mercaptoalkyle, alkylthioalkyle, alkylsulfinylalkyle, alkylsulfonylalkyle, carboxyalkyle, alkoxycarbonylalkyle, arylalkoxycarbonylalkyle, carbamoylalkyle, aminoalkyle, alkylaminoalkyle, dialkylaminoalkyle, guanidinoalkyle, qui peut être substitué le cas échéant par un ou deux restes benzyloxycarbonyle ou par un, deux, trois ou quatre restes alkyle, un groupe alkoxycarbonylaminoalkyle, 9-fluorénylméthoxycarbonyl(Fmoc)aminoalkyle, alcényle, cycloalkyle, cycloalkylalkyle ainsi qu'un groupe arylalkyle éventuellement substitué, les substituants mentionnés étant des radicaux halogéno, hydroxy, alkyle, alkoxy,
R³, R⁶, R⁹ et R¹² représentent, indépendamment les uns des autres, l'hydrogène, un groupe alkyle linéaire ou ramifié ayant jusqu'à 8 atomes de carbone, un groupe hydroxyalkyle, alcanoyloxyalkyle, alkoxyalkyle, aryloxyalkyle, alkylthioalkyle, alkylsulfinylalkyle, alkylsulfonylalkyle, carboxyalkyle, alkoxycarbonylalkyle, arylalkoxycarbonylalkyle, carbamoylalkyle, aminoalkyle, alkylaminoalkyle, dialkylaminoalkyle, alkoxycarbonylaminoalkyle, alcényle, cycloalkyle, cycloalkylalkyle, ainsi qu'un groupe aryle, hétaryle, arylalkyle ou hétarylalkyle éventuellement substitué, avec comme substituants des radicaux halogéno, hydroxy, alkyle, alkoxy, nitro ou 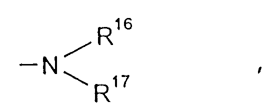 chaque reste pouvant être de l'hydrogène, un reste alkoxy, ou bien les deux restes pouvant former ensemble un noyau pentagonal, hexagonal ou heptagonal qui est éventuellement interrompu par O, S ou N et qui est substitué le cas échéant avec un radical alkyle en C₁ à C₄,
B est un groupe hydroxy, alkoxy ayant jusqu'à 4 atomes de carbone ou le reste NR¹³R¹⁴, dans lequel
R¹³ et R¹⁴ représentent de l'hydrogène, des radicaux alkyle, aralkyle ou aryle,
ainsi que de leurs isomères optiques et leurs racémates, pour la préparation de compositions destinées à combattre des endoparasites en médecine humaine et en médecine vétérinaire.

2. Compositions endoparasiticides, caractérisées par une teneur en au moins un composé de formule (I) suivant la revendication 1.

3. Procédé de préparation de compositions endoparasiticides, caractérisé en ce qu'on mélange des composés de formule (I) suivant la revendication 1 avec des diluants et/ou des agents tensio-actifs.

4. Utilisation d'octadepsipeptides à chaîne ouverte de formule (I) suivant la revendication 1, dans laquelle
A représente de l'hydrogène, un groupe alkyle en C₁ à C₄ ou benzyle,
ou un groupe de formule -CO-R¹⁵,
dans laquelle
R¹⁵ est un groupe alkyle en C₁ à C₄, alcényle en C₂ à C₄, alkoxy en C₁ à C₄ à chaîne droite ou ramifiée ou un groupe phénylalkoxy ayant jusqu'à 6 atomes de carbone dans la partie alkyle,
R¹, R⁴, R⁷ et R¹⁰ représentent, indépendamment les uns des autres, un groupe alkyle en C₁ à C₄, cycloalkyle en C₃ à C₆, phényl-(alkyle en C₁ à C₄),
R², R⁵, R⁸ et R¹¹ représentent, indépendamment les uns des autres, l'hydrogène, un groupe, linéaire ou ramifié, alkyle en C₁ à C₈, hydroxyalkyle en C₁ à C₆, (alcanoyloxy en C₁ à C₄)-(alkyle en C₁ à C₆), (alkoxy en C₁ à C₄)-(alkyle en C₁ à C₆), aryl-(alkyloxy en C₁ à C₄)-(alkyle en C₁ à C₆), mercapto-alkyle en C₁ à C₆, (alkylthio en C₁ à C₄)-(alkyle en C₁ à C₆), (alkylsulfinyle en C₁ à C₄)-(alkyle en C₁ à C₆), (alkylsulfonyle en C₁ à C₄)-(alkyle en C₁ à C₆), carboxy-(alkyle en C₁ à C₆), (alkoxy en C₁ à C₄)carbonyl-(alkyle en C₁ à C₆, aryl-(alkoxy en C₁ à C₄)carbonyl-(alkyle en C₁ à C₆), carbamoyl-(alkyle en C₁ à C₆), aminoalkyle en C₁ à C₆, (alkyle en C₁ à C₄)amino-(alkyle en C₁ à C₆), di-(alkyle en C₁ à C₄)-amino-(alkyle en C₁ à C₆), guanido-(alkyle en C₁ à C₆), (alkoxy en C₁ à C₄)carbonylamino-(alkyle en C₁ à C₆), alcényle en C₂ à C₈), cycloalkyle en C₃ à C₇, (cycloalkyle en C₃ à C₇)-(alkyle en C₁ à C₄), phényl-(alkyle en C₁ à C₄), qui peut être substitué le cas échéant par des restes de la série halogène, alkoxy en C₁ à C₄, alkyle en C₁ à C₄,
R³, R⁶, R⁹ et R¹² représentent, indépendament les uns des autres, de l'hydrogène, un groupe, linéaire ou ramifié, alkyle en C₁ à C₈, hydroxyalkyle en C₁ à C₆, (alcanoyloxy en C₁ à C₄)-(alkyle en C₁ à C₆), (alkoxy en C₁ à C₄)-(alkyle en C₁ à C₆), aryl-(alkyloxy en C₁ à C₄)-(alkyle en C₁ à C₆), mercapto-alkyle en C₁ à C₆, (alkylthio en C₁ à C₄)-(alkyle en C₁ à C₆), (alkylsulfinyle en C₁ à C₄)-(alkyle en C₁ à C₆), (alkylsulfonyle en C₁ à C₄)-(alkyle en C₁ à C₆), carboxy-(alkyle en C₁ à C₆), (alkoxy en C₁ à C₄)-carbonyl-(alkyle en C₁ à C₆), aryl-(alkoxy en C₁ à C₄)-carbonyl-(alkyle en C₁ à C₆), carbamoyl-(alkyle en C₁ à C₆), amino-alkyle en C₁ à C₆, (alkyle en C₁ à C₄)-amino-(alkyle en C₁ à C₆), di-(alkyle en C₁ à C₄)amino-(alkyle en C₁ à C₆), guanidino-(alkyle en C₁ à C₆), (alkoxy en C₁ à C₄)carbonylamino-(alkyle en C₁ à C₆), alcényle en C₂ à C₈, cycloalkyle en C₃ à C₇, (cycloalkyle en C₃ à C₇)-(alkyle en C₁ à C₄), aryl-(alkyle en C₁ à C₄) ou hétaryl-(alkyle en C₁ à C₄), qui peuvent être substitués le cas échéant par des restes de la série halogène, hydroxy, alkoxy en C₁ à C₄, alkyle en C₁ à C₄, nitro ou un groupe amino de formule 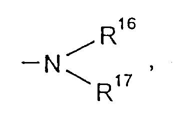 dans lequel chaque reste R¹⁶ ou R¹⁷ représente de l'hydrogène, un groupe alkyle en C₁ à C₄, alkoxy en C₁ à C₄, ou bien les deux restes peuvent former conjointement un noyau pentagonal, hexagonal ou heptagonal qui est interrompu, le cas échéant, par O, S ou N et qui est éventuellement substitué par un groupe alkyle en C₁ à C₄,
B est un groupe hydroxy, tertio-butoxy ou le reste NR¹³R¹⁴, dans lequel
R¹³ et R¹⁴ représentent, indépendamment l'un de l'autre, l'hydrogène, un groupe alkyle en C₁ à C₄ linéaire ou ramifié, benzyle, cyclopropyle, cyclohexyle, phényle,
ainsi que de leurs isomères optiques et de leurs racémates.

5. Utilisation d'octadepsipeptides à chaîne ouverte de formule (I) suivant la revendication 1 dans laquelle
A représente l'hydrogène, un groupe benzyle ou un groupe COR¹⁵,
R¹⁵ est un groupe alkoxy ou aryloxy linéaire ou ramifié ayant jusqu'à 6 atomes de carbone dans la partie alkyle,
R¹, R⁴, R⁷ et R¹⁰ représentent, indépendamment les uns des autres, des groupes alkyle en C₁ à C₄, cycloalkyle en C₃ à C₆ et benzyle,
R², R⁵, R⁸ et R¹¹ représentent, indépendamment les uns des autres, de l'hydrogène, un groupe, linéaire ou ramifié, alkyle en C₁ à C₈, (alcanoyloxy en C₁ à C₄)-(alkyle en C₁ à C₆), (alkoxy en C₁ à C₄)-(alkyle en C₁ à C₆), aryl-(alkyloxy en C₁ à C₄)-(alkyle en C₁ à C₆, (alkoxy en C₁ à C₄)-carbonylamino-(alkyle en C₁ à C₆), alcényle en C₂ à C₈, un groupe cycloalkyle en C₃ à C₇, (cycloalkyle en C₃ à C₇)-(alkyle en C₁ à C₄), phényl-(alkyle en C₁ à C₄), qui peut être substitué le cas échéant par un ou plusieurs restes, identiques ou différents, de la série halogéno, méthoxy, éthoxy, méthyle,
R³, R⁶, R⁹ et R¹² représentent, indépendamment les uns des autres, de l'hydrogène, un groupe, linéaire ou ramifié, alkyle en C₁ à C₈, (alcanoyloxy en C₁ à C₄-(alkyle en C₁ à C₆), (alkoxy en C₁ à C₄)-(alkyle en C₁ à C₆), aryl-(alkyloxy en C₁ à C₄)-(alkyle en C₁ à C₆), (alkoxy en C₁ à C₄)-carbonylamino-(alkyle en C₁ à C₆), alcényle en C₂ à C₈, un groupe cycloalkyle en C₃ à C₇, (cycloalkyle en C₃ à C₇)-(alkyle en C₁ à C₄), aryl-(alkyle en C₁ à C₄) ou hétaryl-(alkyle en C₁ à C₄), qui peut être substitué le cas échéant par un ou plusieurs restes, identiques ou différents, de la série halogéno, méthoxy, éthoxy, nitro ou un groupe amino de formule -NR¹⁶R¹⁷ dans laquelle R¹⁶ et R¹⁷ ont la définition indiquée dans la revendication 4,
B est un groupe hydroxy ou tertio-butoxy,
ainsi que de leurs isomères optiques et leurs racémates.

6. Utilisation d'octadepsipeptides à chaîne ouverte de formule (I) suivant la revendication 1, dans laquelle
A représente de l'hydrogène ou un groupe benzyle,
R¹, R⁴, R⁷ et R¹⁰ représentent, indépendamment les uns des autres, des groupes méthyle, éthyle, propyle, et isopropyle,
R², R⁵, R⁸ et R¹¹ représentent, indépendamment les uns des autres, de l'hydrogène, des groupes méthyle, éthyle, propyle, isopropyle, butyle, isobutyle, sec.-butyle, pentyle, isopentyle, sec.-pentyle, hexyle, isohexyle, sec.-hexyle, heptyle, isoheptyle, sec.-heptyle, octyle, isooctyle, sec.-octyle, allyle, cyclohexylméthyle, phénylméthyle,
R³, R⁶, R⁹ et R¹² représentent, indépendamment les uns des autres, l'hydrogène, des groupes méthyle, éthyle, propyle, isopropyle, butyle, isobutyle, sec.-butyle, pentyle, isopentyle, sec.-pentyle, hexyle, isohexyle, sec.-hexyle, heptyle, isoheptyle, sec.-heptyle, octyle, isooctyle, sec.-octyle, vinyle, allyle, cyclohexylméthyle, aryl-(alkyle en C₁ à C₄), ou hétaryl-(alkyle en C₁ à C₄) qui peut être substitué le cas échéant par un ou plusieurs restes, identiques ou différents, indiqués dans la revendication 4,
B est un groupe hydroxy ou tertio-butoxy,
ainsi que de leurs isomères optiques et leurs racémates.
